# EUROPEAN PATENT APPLICATION

(11) **EP 2 703 400 A1**
(43) Date of publication of application: **05.03.2014**
(21) Application number: 12777084.0
(22) Date of filing: 26.04.2012
(51) Int. Cl.: C07D 413/14, A61K 31/497, A61P 3/10

(54) **PHENYL PYRROLE DERIVATIVE CRYSTAL**

(30) Priority: 27.04.2011 JP 2011100162
(71) Applicant: Daiichi Sankyo Company, Limited, Tokyo 103-8426 (JP)
(72) Inventor: ONO, Makoto, Tokyo 140-8710 (JP)
(74) Representative: Fairbairn, Angus Chisholm
(86) International application number: PCT/JP2012/061175
(87) International publication number: WO 2012/147832

(57) **Abstract**

Provided is a monomethanesulfonate of a phenylpyrrole derivative, having superior glucokinase activating activity and demonstrating remarkably improved solubility, hygroscopicity and stability as well as superior oral absorption, and crystals thereof. The present invention provides (2S)-2-(3-{5-[(5S)-5-methyl-4,5-dihydro-1,3-oxazol-2-yl]-1H-pyrrol-2-yl}-5-{[5-(methylsulfonyl)pyrazin-2-yl]oxy}phenoxy)propan-1-ol monomethanesulfonate having superior glucokinase activating activity, crystals thereof, a pharmaceutical containing the same, and a preventive and/or therapeutic agent for diabetes and the like.

## Description

### TECHNICAL FIELD

The present invention relates to a crystal of a compound that has superior glucokinase activating activity and is useful as a therapeutic for diabetes and the like.

### BACKGROUND ART

Substances having glucokinase (abbreviated as GK in the present description) activating activity are known to be useful as diabetes or impaired glucose tolerance therapeutics and preventives, or as therapeutics and preventives for chronic complications of diabetes, including diabetic retinopathy, diabetic nephropathy, diabetic neuropathy, ischemic heart disease and arteriosclerosis.

(2S)-2-(3-(5-[(5S)-5-Methyl-4,5-dihydro-1,3-oxazol-2-yl]-1H-pyrrol-2-yl}-5-{[5-(methylsulfonyl)pyrazin-2-yl]oxy}phenoxy)propan-1-ol having a phenylpyrrole site has been reported to be a substance that has GK activating activity (Patent Document 1).
Patent Document 1: International Publication No. WO 2009/099080 Pamphlet

### DISCLOSURE OF THE INVENTION

### Problems to be Solved by the Invention

An object of the present invention is to provide a therapeutic and preventive for diabetes and impaired glucose tolerance in particular by forming a crystal of a GK activator. As a result of conducting extensive studies on compounds having GK activating activity, the inventor of the present invention found that superior pharmacological activity is demonstrated by converting a known phenylpyrrole compound to a methanesulfonate crystal.

Namely, a novel crystal of (25)-2-(3-{5-[(5S)-5-methyl-4,5-dihydro-1,3-oxazol-2-yl]-1H-pyrrol-2-yl}-5-{[5-(methylsulfonyl)pyrazin-2-yl]oxy}phenoxy)propan-1-ol monomethanesulfonate (also referred to as "Compound I" in the present description), obtained by converting (2S)-2-(3-{5-[(5S)-5-methyl-4,5-dihydro-1, 3-oxazol-2-yl]-1H-pyrrol-2-yl}-5-([5-(methylsulfonyl)pyrazin-2-yl]oxy}phenoxy)propan-1-ol which is a phenylpyrrole compound to a methanesulfonate, has remarkably improved solubility and pharmacokinetics in comparison with the free form thereof (compound that is not formed as a salt) and demonstrates superior oral absorption. Due to this superior oral absorption, an adequate concentration in the blood can be secured and superior pharmacological activity can be obtained with a smaller amount. In addition, bioavailability (BA) is also superior.

### Means for Solving the Problems

The present invention relates to:
(1) (2S)-2-(3-{5-[(5S)-5-methyl-4,5-dihydro-1,3-oxazol-2-yl]-1H-pyrrol-2-yl}-5-{[5-(methylsulfonyl)pyrazin-2-yl]oxy}phenoxy)propan-1-ol methanesulfonate.
Moreover, the present invention relates to:
(2) (2S)-2-(3-{5-[(5S)-5-methyl-4,5-dihydro-1,3-oxazol-2-yl]-1H-pyrrol-2-yl}-5-{[5-(methylsulfonyl)pyrazin-2-yl]oxy}phenoxy)propan-1-ol monomethanesulfonate represented by the following formula (I). 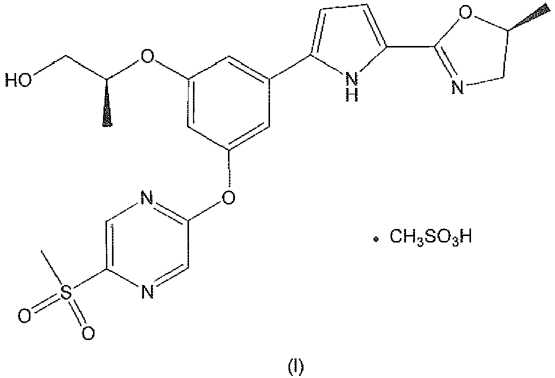
(3) A crystal of (2S)-2-(3-{5-[(5S)-5-methyl-4,5-dihydro-1,3-oxazol-2-yl]-1H-pyrrol-2-yl}-5-{[5-(methylsulfonyl)pyrazin-2-yl]oxy}phenoxy)propan-1-ol monomethanesulfonate represented by the following formula (I) described in (2). 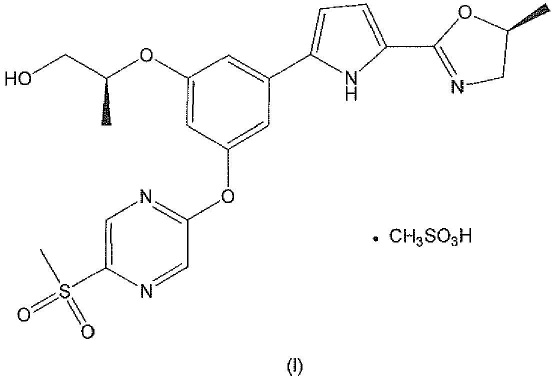
(4) The crystal described in (3), wherein the crystal has characteristic peaks at 8.2, 17.1, 18.0, 19.5, 19.6, 20.0, 20.2, 22.0, 22.2, 23.5 and 24.1 (degrees) (each ±0.2) for the angles of diffraction (2θ) as determined by powder X-ray diffraction obtained by irradiating with Copper Kα radiation.
(5) The crystal described in (3), wherein the crystal has the characteristic peaks and relative intensities (angles of diffraction: ±0.2 each) shown in the following table:

**[Table 1]**

| Angle of diffraction 2θ (degree) | Interplanar spacing d (Å) | Relative intensity (%) |
|---|---|---|
| 8.2 | 10.8 | 15.1 |
| 17.1 | 5.2 | 15.7 |
| 18.0 | 4.9 | 15.5 |
| 19.5 | 4.6 | 28.8 |
| 19.6 | 4.5 | 17.2 |
| 20.0 | 4.4 | 22.2 |
| 20.2 | 4.4 | 100 |
| 22.0 | 4.0 | 42.7 |
| 22.2 | 4.0 | 20.6 |
| 23.5 | 3.8 | 25.1 |
| 24.1 | 3.7 | 15.4 |

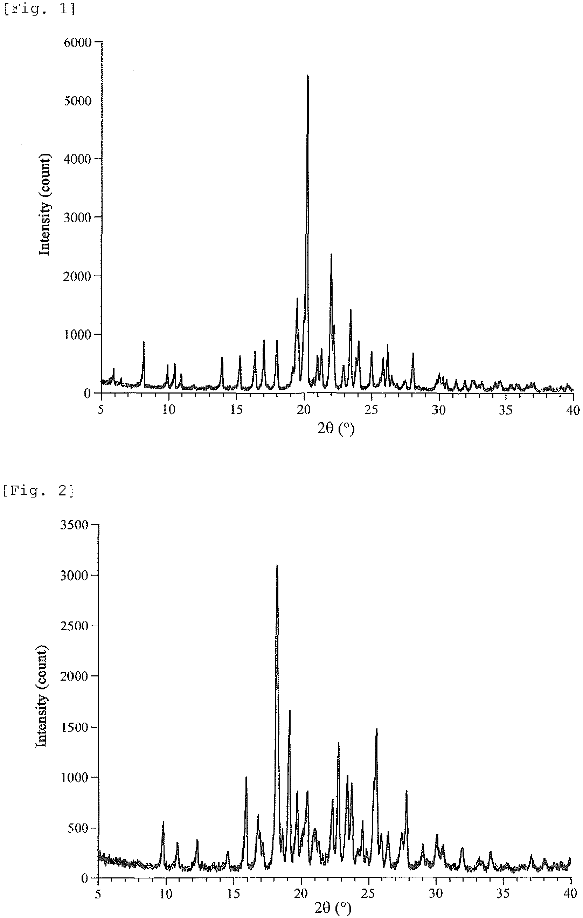

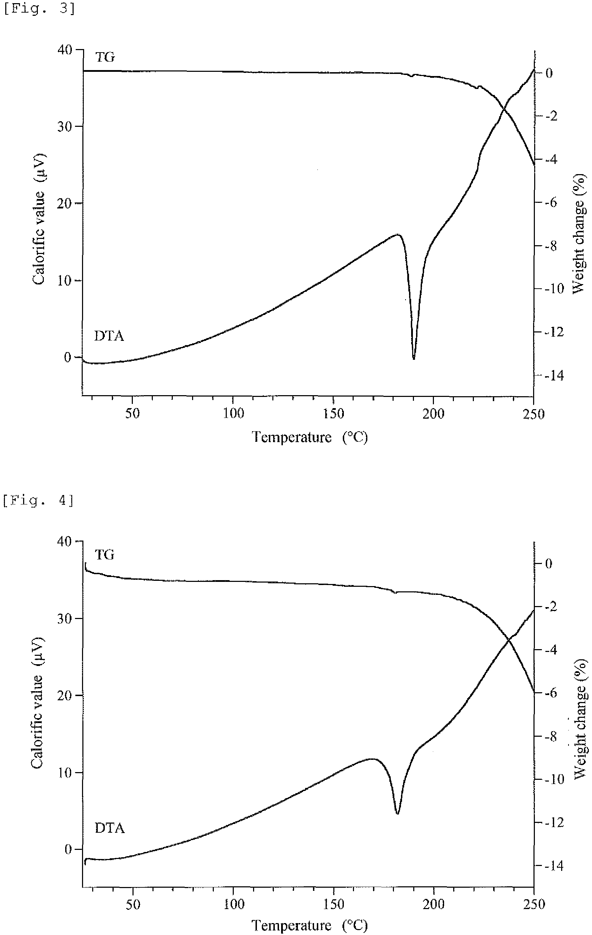

for the angles of diffraction (2θ) as determined by powder X-ray diffraction obtained by irradiating with Copper Kα radiation.
(6) The crystal described in (3), wherein the crystal has the characteristic peaks indicated by the pattern shown in FIG. 1 for the angles of diffraction (2θ) as determined by powder X-ray diffraction obtained by irradiating with Copper Kα radiation.
(7) The crystal described in (3), wherein the crystal has at least one endothermic peak at 185°C to 195°C in differential thermal analysis (DTA).
(8) The crystal described in (3), wherein the crystal has the characteristic peaks indicated by the pattern shown in FIG. 3 as a thermogravimetry-differential thermal analysis (TG/DTA) profile.
(9) The crystal described in (3), wherein the crystal has characteristic peaks at 9.8, 15.9, 16.8, 18.2, 19.1, 19.7, 20.5, 22.3, 22.8, 23.4, 23.8, 24.6, 25.4, 25.6 and 27.8 (degrees) (each ±0.2) for the angles of diffraction (2θ) as determined by powder X-ray diffraction obtained by irradiating with Copper Kα radiation.
(10) The crystal described in (3), wherein the crystal has the characteristic peaks and relative intensities (angles of diffraction: ±0.2 each) shown in the following table:

**[Table 2]**

| Angle of diffraction 2θ (degree) | Interplanar spacing d (Å) | Relative intensity (%) |
|---|---|---|
| 9.8 | 9.0 | 15.3 |
| 15.9 | 5.6 | 31.2 |
| 16.8 | 5.3 | 18.4 |
| 18.2 | 4.9 | 100 |
| 19.1 | 4.6 | 53.7 |
| 19.7 | 4.5 | 26.1 |
| 20.5 | 4.3 | 26.4 |
| 22.3 | 4.0 | 22.9 |
| 22.8 | 3.9 | 42.6 |
| 23.4 | 3.8 | 31.7 |
| 23.8 | 3.7 | 29.3 |
| 24.6 | 3.6 | 16.2 |
| 25.4 | 3.5 | 28.5 |
| 25.6 | 3.5 | 47.6 |
| 27.8 | 3.2 | 26.4 |

for the angles of diffraction (2θ) as determined by powder X-ray diffraction obtained by irradiating with Copper Kα radiation.
(11) The crystal described in (3), wherein the crystal has the characteristic peaks indicated by the pattern shown in FIG. 2 for the angles of diffraction (2θ) as determined by powder X-ray diffraction obtained by irradiating with Copper Kα radiation.
(12) The crystal described in (3), wherein the crystal has at least one endothermic peak at 175°C to 185°C in differential thermal analysis (DTA).
(13) The crystal described in (3), wherein the crystal has the characteristic peaks indicated by the pattern shown in FIG. 4 as a thermogravimetry-differential thermal analysis (TG/DTA) profile.
(14) A pharmaceutical composition containing as an active ingredient thereof a compound described in (1) or (2) or a crystal described in any one selected from (3) to (13).
(15) A pharmaceutical composition containing as an active ingredient thereof a crystal described in any one selected from (4) to (8) and/or a crystal described in any one selected from (9) to (13).
(16) The pharmaceutical composition described in (14) or (15), wherein the pharmaceutical composition is for treating and/or preventing diabetes or impaired glucose tolerance.
(17) A preventive drug or therapeutic drug for diabetes or impaired glucose tolerance containing as an active ingredient thereof a compound described in (1) or (2) or a crystal described in any one selected from (3) to (13).
(18) Use of a compound described in (1) or (2) or a crystal described in any one selected from (3) to (13) for a preventive drug or therapeutic drug for diabetes or impaired glucose tolerance.
(19) A method for producing a compound described in (2), characterized by reacting methanesulfonic acid with (2S)-2-(3-{5-[(5S)-5-methyl-4,5-dihydro-1,3-oxazol-2-yl]-1H-pyrrol-2-yl}-5-{[5-(methylsulfonyl)pyrazin-2-yl]oxy}phenoxy)propan-1-ol in a solvent.
(20) The method described in (19), wherein the solvent is aqueous acetone.
(21) The method described in (19), wherein the solvent is aqueous 1-propanol.

The (2S)-2-(3-{5-[(5S)-5-methyl-4,5-dihydro-1,3-oxazol-2-yl]-1H-pyrrol-2-yl}-5-{[5-(methylsulfonyl)pyrazin-2-yl]oxy}phenoxy)propan-1-ol methanesulfonate of the present invention refers to a compound in which (2S)-2-(3-{5-[(5S)-5-methyl-4,5-dihydro-1,3-oxazol-2-yl]-1H-pyrrol-2-yl}-5-{[5-(methylsulfonyl)pyrazin-2-yl]oxy}phenoxy)propan-1-ol and methanesulfonic acid are ionically bonded. Although varying depending on the conditions under which both are allowed to react, the ratio of both is such that all compounds in which they are ionically bonded at any arbitrary ratio are included. The compounds are preferably ionically bonded at a ratio of 1:1, namely in the form of (2S)-2-(3-{5-[(5S)-5-methyl-4,5-dihydro-1,3-oxazol-2-yl]-1H-pyrrol-2-yl}-5-{[5-(methylsulfonyl)pyrazin-2-yl]oxy}phenoxy)propan-1-ol monomethanesulfonate.

A crystal of Compound I of the present invention indicates a solid in which the internal structure thereof is three-dimensionally composed of an orderly repetition of constituent atoms (or groups thereof), and is distinguished from an amorphous solid not having this type of orderly internal structure. Whether or not a solid is crystalline can be investigated by well-known crystallographic methods (such as measurement by powder X-ray diffraction or differential scanning calorimetry). For example, in the case of measuring by powder X-ray diffraction using X-rays obtained by irradiating a solid with Copper Kα radiation, and well-defined peaks are observed in the resulting X-ray diffraction diagram, the solid is determined to be crystalline, while in the case that well-defined peaks are not observed, the solid is determined to be amorphous. In the case that peaks can be read but are not well-defined (such as in the case of broad peaks), the solid is determined to consist of crystals having a low degree of crystallinity, and such crystals having a low degree of crystallinity are also included in the crystal of the present invention.

Even in the case of crystals of the same compound, a plurality of crystals having different internal structures and physicochemical properties may be formed depending on the crystallization conditions (crystal polymorphism), and the crystal of the present invention may be any of these crystals or mixtures of two or more thereof. Thus, the crystal of the present invention includes these crystals as well as all mixtures of these crystals at any ratio.

The crystal of the present invention may have adhered water by adsorbing moisture as a result of being allowed to stand in air, or may form hydrates such as by heating to 25°C to 150°C under ordinary atmospheric conditions. Moreover, the crystal of the present invention may also contain a solvent used during crystallization as adhered residual solvent or as a solvate.

Physical properties of the resulting crystals can be investigated using a powder X-ray diffraction analyzer or various other instruments useful for analyzing crystals, such as an infrared spectrometer, thermogravimetry differential thermal analyzer (TG/TDA) or water vapor sorption analyzer.

In the present description, although the crystals of the present invention can be represented on the basis of powder X-ray diffraction data, powder X-ray diffraction measurements and analysis may be carried out in accordance with usual techniques used in the relevant field, and can be carried out by, for example, the methods described in the section on test examples. In addition, since the lattice constants of hydrates and dehydrates typically change due to adsorption and desorption of water of crystallization, this can result in a change in angle of diffraction (2θ) in powder X-ray diffraction. In addition, peak intensity also varies according to differences in crystal growth face etc. (crystal habit) and the like. Thus, in the case of representing the crystals of the present invention on the basis of powder X-ray diffraction data, crystals for which angles of diffraction peaks and X-ray diffraction diagrams agree in powder X-ray diffraction as well as hydrates and dehydrates obtained therefrom are included in the scope of the present invention.

During powder diffraction measurement using Copper Kα radiation, a sample is normally irradiated with Copper Kα radiation (those for which the Kα1 X-ray and the Kα2 X-ray have not been separated). X-ray diffraction diagrams can be obtained by analyzing the diffraction attributable to Kα X-rays, and can also be obtained by analyzing only the diffraction attributable to the Kα1 X-ray extracted from the diffraction attributable to Kα X-rays. In the present invention, powder X-ray diffraction diagrams obtained by irradiating with Kα X-rays include X-ray diffraction diagrams obtained by analyzing diffraction peaks attributable to Kα X-rays as well as X-ray diffraction diagrams obtained by analyzing diffraction attributable to the Kα1 X-ray, and are preferably X-ray diffraction diagrams obtained by analyzing diffraction attributable to the Kα1 X-ray.

Examples of the crystals of Compound I of the present invention include crystals having characteristic peaks at angles of diffraction 2θ (degrees) of 8.2, 17.1, 18.0, 19.5, 19.6, 20.0, 20.2, 22.0, 22.2, 23.5 and 24.1, and crystals having characteristic peaks at angles of diffraction 2θ (degrees) of 9.8, 15.9, 16.8, 18.2, 19.1, 19.7, 20.5, 22.3, 22.8, 23.4, 23.8, 24.6, 25.4, 25.6 and 27.8, as determined by powder X-ray diffraction obtained by irradiating with Copper Kα radiation. Here, "characteristic peaks" refers to peaks having a relative intensity of 15 or more based on a value of 100 for the maximum peak intensity in powder X-ray diffraction.

In the powder X-ray diffraction diagrams shown in Figs. 1 and 2, diffraction intensity (counts/sec (cps)) is represented on the vertical axis, while angle of diffraction 2θ (degrees) is represented on the horizontal axis. Since the location and relative intensity of the angle of diffraction 2θ can vary somewhat according to measurement conditions and the like, the identity of the crystal form may be confirmed by suitably referring to the entire spectral pattern if the angle of diffraction 2θ varies slightly. That error is normally within the range of ±2, preferably within the range of ±1, more preferably within the range of ±0.5, and even more preferably within the range of +0.2.

In addition, the intensity of each diffraction peak can also vary due to numerous factors (including the effects of preferred orientation and particle size attributable to a specific crystal form) as is commonly known in the field of crystallography, and although the relative intensities of the aforementioned main peaks for specifying the crystal of the present invention can also vary, these crystals are also included in the crystal of the present invention.

### EFFECTS OF THE INVENTION

According to the present invention, a crystal of a phenylpyrrole derivative can be provided that has superior solubility, hygroscopicity and stability. A crystal of a sulfonate of the phenylpyrrole derivative of the present invention is effective as a preventive and/or therapeutic for diabetes or impaired glucose tolerance by activating GK.

### BRIEF DESCRIPTION OF THE DRAWINGS

Fig. 1 is a powder X-ray diffraction diagram of the crystal obtained in Example 1 in which the vertical axis of the diagram represents diffraction intensity in units of counts/sec (cps) and the horizontal axis represents angle of diffraction 2θ.
Fig. 2 is a powder X-ray diffraction diagram of the crystal obtained in Example 2 in which the vertical axis of the diagram represents diffraction intensity in units of counts/sec (cps) and the horizontal axis represents angle of diffraction 2θ.
Fig. 3 is a thermogravimetry-differential thermal analysis (TG/DTA) pattern diagram of the crystal obtained in Example 1 in which the vertical axis of the diagram represents calorific value (µV) or weight change (%) and the horizontal axis represents temperature (°C), and which indicates an endothermic peak in the vicinity of 190°C.
Fig. 4 is a thermogravimetry-differential thermal analysis (TG/DTA) pattern diagram of the crystal obtained in Example 2 in which the vertical axis of the diagram represents calorific value (µV) or weight change (%) and the horizontal axis represents temperature (°C), and which indicates an endothermic peak in the vicinity of 182°C.
Fig. 5 is a diagram indicating the moisture sorption-desorption behavior of the crystal obtained in Example 1 in which the vertical axis of the diagram represents weight change (%) and the horizontal axis represents relative humidity (%).
Fig. 6 is a diagram indicating the moisture sorption-desorption behavior of the crystal obtained in Example 2 in which the vertical axis of the diagram represents weight change (%) and the horizontal axis represents relative humidity (%).

### MODE FOR CURRYING OUT THE INVENTION

(2S)-2-(3-{5-[(5S)-5-Methyl-4,5-dihydro-1,3-oxazol-2-yl]-1H-pyrrol-2-yl}-5-{[5-(methylsulfonyl)pyrazin-2-yl]oxy}phenoxy)propan-1-ol (also referred to as "Compound II" in the present description), represented by the following formula (II): 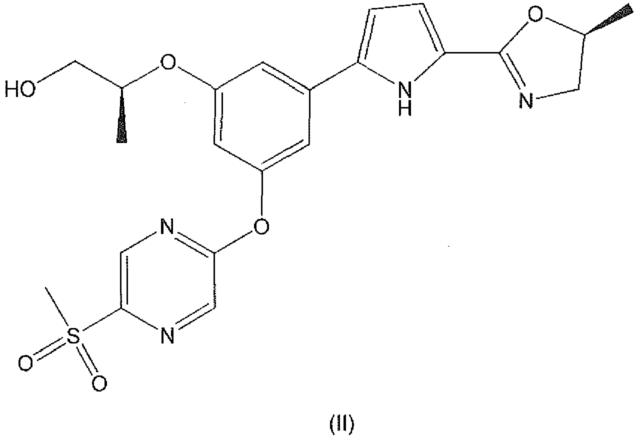 is the free form of Compound I.

There are no particular limitations on the method used to produce Compound II, and it can be produced using, for example, the method described in Patent Document 1 or a method in compliance therewith.

There are no particular limitations on the method used to produce Compound I, and Compound I can be obtained in the form of a crystalline compound by, for example, allowing methanesulfonic acid to react with Compound II in a solvent and precipitating the crystal.

The solvent used is preferably methanol, ethanol, 1-propanol, 2-propanol, acetone, acetonitrile, tetrahydrofuran, dioxane or a hydrous solvent thereof, and is more preferably aqueous acetone and aqueous 1-propanol.

The level of water content in the hydrous solvent is normally 3% to 12.5%. The level of water content in aqueous acetone is preferably 4% to 10% and more preferably 5%. The level of water content in aqueous 1-propanol is preferably 5% to 12% and more preferably 10%.

The temperature is normally 15°C to 40°C and preferably 20°C to 25°C.

The compound or crystal thereof of the present invention can be administered in various forms. Examples of the route of administration include oral administration using tablets, capsules, granules, emulsions, pills, powders, syrups (solutions), and the like and parenteral administration using injections (intravenous, intramuscular, subcutaneous, or intraperitoneal administration), drip infusions, suppositories (rectal administration), and the like. These various formulations can be prepared as drug products according to usual methods using aids usually used in the field of drug formulation such as excipients, binders, disintegrants, lubricants, flavoring agents, dissolving aids, suspending agents, and coating agents in addition to the active ingredient.

In the use as a tablet, examples of carriers that can be used include excipients such as lactose, sucrose, sodium chloride, glucose, urea, starch, calcium carbonate, kaolin, crystalline cellulose, and silicic acid; binders such as water, ethanol, propanol, simple syrup, glucose solution, starch solution, gelatin solution, carboxymethylcellulose, shellac, methylcellulose, potassium phosphate, and polyvinylpyrrolidone; disintegrants such as dry starch, sodium alginate, agar powder, laminaran powder, sodium hydrogencarbonate, calcium carbonate, polyoxyethylene sorbitan fatty acid esters, sodium lauryl sulfate, stearic monoglyceride, starch, and lactose; disintegration inhibitors such as sucrose, stearin, cocoa butter, and hydrogenated oil; absorption enhancers such as quaternary ammonium salts and sodium lauryl sulfate; humectants such as glycerine and starch; adsorbents such as starch, lactose, kaolin, bentonite, and colloidal silicic acid; lubricants such as purified talc, stearate, boric acid powder, and polyethylene glycol, and so forth. Furthermore, tablets coated in usual ways such as, for example, sugar-coated tablets, gelatin-coated tablets, enteric-coated tablets, film-coated tablets, double-layer tablets, and multilayered tablets can be prepared as required.

In the use as a pill, examples of carriers that can be used include excipients such as glucose, lactose, cocoa butter, starch, hydrogenated vegetable oil, kaolin, and talc; binders such as powdered gum arabic, powdered tragacanth, gelatin, and ethanol; disintegrants such as laminaran, and agar, and so forth.

In the use as a suppository, a wide range of carriers conventionally known in this field can be used, and examples thereof include polyethylene glycol, cocoa butter, higher alcohols, higher alcohol esters, gelatin, semisynthetic glycerides, and so forth.

In the use as an injection, the formulations can be prepared as solutions, emulsions, or suspensions. Preferably, these solutions, emulsions, and suspensions are sterilized and are isotonic with blood. Solvents for producing these solutions, emulsions, and suspensions are not particularly limited so long as they can be used as diluents for medical use, and examples thereof include water, ethanol, propylene glycol, ethoxylated isostearyl alcohol, polyoxylated isostearyl alcohol, polyoxy ethylene sorbitan fatty acid esters, and so forth. In this case, a sufficient amount of sodium chloride, glucose, or glycerine may be contained in the formulation to prepare an isotonic solution, and usual dissolving aids, buffers, soothing agents, and the like may also be contained therein.

Furthermore, coloring agents, preservatives, perfumes, flavoring agents, sweeteners, and the like can be added to the above-mentioned formulation, if necessary. Furthermore, other drugs can also be added.

The amount of active ingredient compound contained in the above-mentioned formulations is not particularly limited, but is usually 0.5 to 70% by weight of the total composition, preferably 1 to 30% by weight.

The dosage varies depending on symptoms, age, and the like of the patient (a warm-blooded animal, in particular, a human). In the case of oral administration, the recommended adult daily dosage is from 0.1 mg as the lower limit (preferably 1 mg, more preferably 10 mg) to 2000 mg as the upper limit (preferably 100 mg), which is desirably administered by dividing into 1 to 6 doses depending on the symptoms.

### EXAMPLES

Although the following provides a more detailed explanation of the present invention through examples, test examples and preparation examples, the scope of the present invention is not limited thereby.

### (Example 1)

### (2S)-2-(3-{5-[(5S)-5-Methyl-4,5-dihydro-1,3-oxazol-2-yl]-1H-pyrrol-2-yl}-5-{[5-(methylsulfonyl)pyrazin-2-yl]oxy}phenoxy)propan-1-ol monomethanesulfonate

1.51 g (3.2 mmol) of (2S)-2-(3-{5-[(5S)-5-methyl-4,5-dihydro-1,3-oxazol-2-yl]-1H-pyrrol-2-yl}-5-{[5-(methylsulfonyl)pyrazin-2-yl]oxy}phenoxy)propan-1-ol [the compound disclosed in International Publication No. WO2009/09908 as Example 100] was suspended in 2.0 mL of 5% aqueous acetone, 0.308 g (3.2 mmol) of methanesulfonic acid was added, and 1.6 mL of 5% aqueous acetone was added dropwise with heating to 50-55°C in a water bath. After dissolution, the temperature was brought back to room temperature, followed by stirring for one day. The deposited crystals were filtered off under reduced pressure and then air-dried for one day to afford 1.76 g of the title compound (yield: 96.9%) as a pale yellowish-white crystal.
¹H-NMR (CDCl₃, 400 MHz)
δ: 1.38 (3H, d, J=6.3Hz), 1.68 (3H, d, J=6.3Hz), 3.24 (3H, s), 3.63 (1H, dd, J=5.5, 11.7Hz), 3.78 (1H, dd, J=7.8, 11.3Hz), 3.87 (1H, dd, J=6.3, 11.7Hz), 4.32 (1H, dd, J=9.4, 11.3Hz), 4.79 (1H, dt, J=6.3, 12.1Hz), 5.32-5.41 (1H, m), 6.64 (1H, dd, J=2.3, 4.3Hz), 6.76 (1H, t, J=2.3Hz), 7.13 (1H, dd, J=1.6, 2.3Hz), 7.25 (1H, dd, J=2.3, 4.3Hz), 7.60 (1H, t, J=2.0Hz), 8.50 (1H, d, J=1.6Hz), 8.80 (1H, d, J=1.6Hz), 12.10 (1H, s), 12.61 (1H, s).
Elemental Analysis
C₂₂H₂₄N₄O₆S·CH₃SO₃H
Theoretical C; 48.58, H; 4.96, N; 9.85, O; 25.32, S; 11.28
Found C; 48.59, H; 4.96, N; 9.77, 0; 25.38, S; 11.35
Measuring Equipment
CHN: YANACO TECHNICAL SCIENCE CO. LTD., CHN CORDER MT-6
O: Elementar Corp., vario MICRO cube
S: DIONEX Corp., ICS-1500 Ion Chromatography.

The compound crystal obtained in Example 1 may be referred to as "Form I crystal (Type I crystal)" in the present description.

### (Example 2)

### (2S)-2-(3-{5-[(5S)-5-Methyl-4,5-dihydro-1,3-oxazol-2-yl]-1H-pyrrol-2-yl}-5-{[5-(methylsulfonyl)pyrazin-2-yl]oxy}phenoxy)propan-1-ol monomethanesulfonate

1.00 g (2.1 mmol) of (2S)-2-(3-{5-[(5S)-5-methyl-4,5-dihydro-1,3-oxazol-2-yl]-1H-pyrrol-2-yl}-5-{[5-(methylsulfonyl)pyrazin-2-yl]oxy}phenoxy)propan-1-ol [the compound disclosed in International Publication No. WO2009/099080 as Example 100] was suspended in 1.5 mL of 10% aqueous 1-propanol, 0.208 g (2.1 mmol) of methanesulfonic acid was added, and 0.9 mL of 10% aqueous 1-propanol was added dropwise with heating to 50-55°C in a water bath. After dissolution, the temperature was brought back to room temperature, followed by stirring for one day. The deposited crystals were filtered off under reduced pressure and then air-dried for one day to afford 1.19 g of the title compound (yield: 99.1%) as a pale yellowish-white crystal.
¹H-NMR (CDCl₃, 400 MHz)
δ: 1.37 (3H, d, J=5.9Hz), 1.68 3H, d, J=6.3Hz), 3.24 (3H, s), 3.64 (1H, dd, J=5.1, 11.7Hz), 3.78 (1H, dd, J=8.2, 11.3Hz), 3.86 (1H, dd, J=6.3, 11.7Hz), 4.32 (1H, t, J=9.8Hz), 4.79 (1H, dt, J=6.3, 12.1Hz), 5.33-5.42 (1H, m), 6.64 (1H, dd, J=2.0, 4.3Hz), 6.76 (1H, t, J=2.0Hz), 7.13 (1H, t, J=2.0Hz), 7.25 (1H, dd, J=2.3, 4.3Hz), 7.59 (1H, t, J=2.0Hz), 8.50 (1H, d, J=1.6Hz), 8.80 (1H, d, J=1.2Hz), 12.06 (1H, s), 12.58 (1H, s). Elemental Analysis (including 0.5H₂O of adhesion water) C₂₂H₂₄N₄O₆S·CH₃SO₃H·0.5H₂O
Theoretical C; 47.82, H; 5.06, N; 9.70, O; 26.31, S; 11.10
Found C; 47.73, H; 5.10, N; 9.63, O; 26.37, S; 11.29

The compound crystal obtained in Example 2 may be referred to as "Form II crystal (Type II crystal)" in the present description.

### (Test Example 1) Measurement of Powder X-Ray Diffraction

The sample was uniformly placed in a glass sample holder and measured under the following conditions using the X' Pert-MPD PW 3050 (Phillips Corp., proportional counter, equipped with a slit for removing K_{β} rays).

### (Analysis Conditions)

X-ray species: Cu K. (wavelength: 1.54 Å), tube voltage: 40 kV, tube current: 35 mA, scanning rate: 0.02°/sec, steps: 0.01°, scanning range (2θ): 5-40°

### <Measurement Results>

A powder X-ray diffraction diagram obtained by measuring the Form I crystal according to the aforementioned method is shown in Fig. 1. Those peaks having a relative intensity of 15 or more based on a value of 100 for the maximum peak intensity in Fig. 1 are shown in Table 3.

**[Table 3]**

| Angle of diffraction 2θ (degree) | Interplanar spacing d (Å) | Relative intensity (%) |
|---|---|---|
| 8.2 | 10.8 | 15.1 |
| 17.1 | 5.2 | 15.7 |
| 18.0 | 4.9 | 15.5 |
| 19.5 | 4.6 | 28.8 |
| 19.6 | 4.5 | 17.2 |
| 20.0 | 4.4 | 22.2 |
| 20.2 | 4.4 | 100 |
| 22.0 | 4.0 | 42.7 |
| 22.2 | 4.0 | 20.6 |
| 23.5 | 3.8 | 25.1 |
| 24.1 | 3.7 | 15.4 |

A powder X-ray diffraction diagram obtained by measuring the Form II crystal according to the aforementioned method is shown in Fig. 2. Those peaks having a relative intensity of 15 or more based on a value of 100 for the maximum peak intensity in Fig. 2 are shown in Table 4.

**[Table 4]**

| Angle of diffraction 2θ (degree) | Interplanar spacing d (Å) | Relative intensity (%) |
|---|---|---|
| 9.8 | 9.0 | 15.3 |
| 15.9 | 5.6 | 31.2 |
| 16.8 | 5.3 | 18.4 |
| 18.2 | 4.9 | 100 |
| 19.1 | 4.6 | 53.7 |
| 19.7 | 4.5 | 26.1 |
| 20.5 | 4.3 | 26.4 |
| 22.3 | 4.0 | 22.9 |
| 22.8 | 3.9 | 42.6 |
| 23.4 | 3.8 | 31.7 |
| 23.8 | 3.7 | 29.3 |
| 24.6 | 3.6 | 16.2 |
| 25.4 | 3.5 | 28.5 |
| 25.6 | 3.5 | 47.6 |
| 27.8 | 3.2 | 26.4 |

### (Test Example 2) Thermal Analysis Measurement

The sample was weighed in an aluminum pan, and differential thermal measurement and thermogravimetric measurement were carried out simultaneously under the following conditions.

### (Measurement Conditions)

Measuring instrument: TG/DTA6200 (SII Nanotechnology, Inc.)
Sample weight: Approx. 5 mg
Heating temperature: 10°C/min
Measuring range: Room temperature to 300°C
Atmosphere: Flowing nitrogen, 200 ml/min

### (Measurement Results)

The results of measuring the Form I crystal according to the aforementioned method are shown in Fig. 3.

The Form I crystal demonstrated an endothermic peak in the vicinity of 190°C, and weight loss was not observed from room temperature to the vicinity of 150°C.

The results of measuring the Form II crystal according to the aforementioned method are shown in Fig. 4.

The Form II crystal demonstrated an endothermic peak in the vicinity of 182°C, and only slight weight loss was observed from room temperature to the vicinity of 150°C.

### (Test Example 3) Hygroscopicity Test

The sample was weighed in a glass sample cup, and weight was measured under the conditions indicated below.

### (Measurement Conditions)

Measuring instrument: SGA-100 (VTI Corp.)
Measuring humidity: 40, 10, 20, 30, 40, 50, 60, 70, 80, 90, 80, 70, 60, 50, 40, 30, 20 and 10% RH
Measuring temperature: 25°C
Minimum exposure time: 15 min
Maximum exposure time: 120 min
Step transition condition: within 0.03 wt%

The appearance of the sample was observed following completion of measurement.

### (Measurement Results)

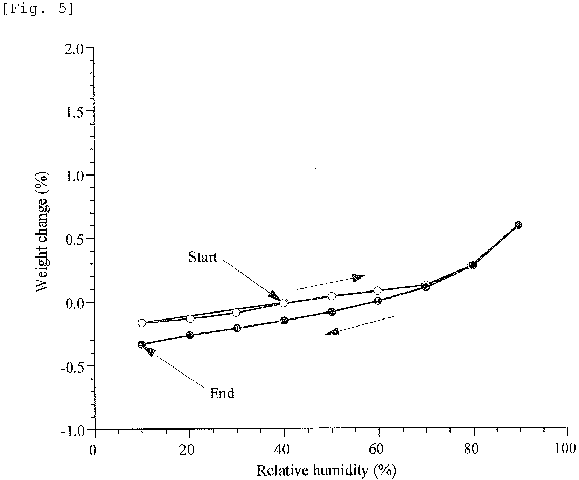

The results of measuring the Form I crystal according to the aforementioned method are shown in Fig. 5.

The Form I crystal did not demonstrate hygroscopicity.

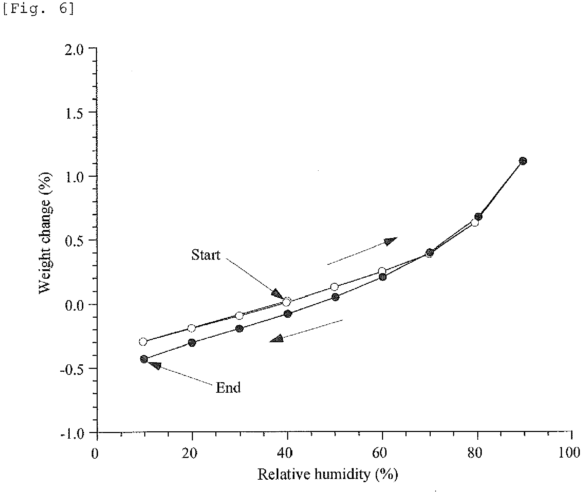

The results of measuring the Form II crystal according to the aforementioned method are shown in Fig. 6.

The Form II crystal did not demonstrate hygroscopicity.

### (Test Example 4) Solubility Test

### (Test Method)

The Form I crystal (4 mg), the Form II crystal (4 mg) and Compound II (4 mg) were respectively dissolved in 2 mL of water, Japanese Pharmacopoeia Elution Test Solution 1 (JP1), Japanese Pharmacopoeia Elution Test Solution 2 (JP2), Fasted State Simulated Intestinal Fluid (FaSSIF) and Fed State Simulated Intestinal Fluid (FeSSIF). After stirring vigorously for 30 seconds every 5 minutes in a constant temperature water bath at 37°C, a portion of the supernatant was sampled 30 minutes later and filtered with a syringe filter. The filtrate was suitably diluted for use as the sample solution.

The sample solution was measured using HPLC under the following conditions.

### (Analysis Conditions)

HPLC system: Waters Alliance
Column: Waters, XBridge C18 3.5 µm
Column size: 3.0 x 50 mm
Column temperature: 40°C
Flow rate: 1.0 mL/min
Solvent A: 5 mM aqueous ammonium hydrogencarbonate solution
Solvent B: Acetonitrile

The gradient program is as indicated below.

**[Table 5]**

| Time (min) | Total flow (mL/min) | % A | % B |
|---|---|---|---|
| 0 | 1.0 | 95 | 5 |
| 10 | 1.0 | 5 | 95 |
| 15 | 1.0 | 5 | 95 |
| 15.1 | 1.0 | 95 | 5 |
| 20 | 1.0 | 95 | 5 |

### (Measurement Results)

As shown in Table 6, the Form I crystal and Form II crystal demonstrated higher solubility in each of the test solutions in comparison with Compound II.

**[Table 6]**

| Test Solution | Concentration (µg/mL) | | |
|---|---|---|---|
| | Compound II | Form I crystal | Form II crystal |
| Water | 5.59 | >1500 | >1500 |
| Japanese Pharmacopoeia Elution Test Solution 1 | >1500 | >1500 | >1500 |
| Japanese Pharmacopoeia Elution Test Solution 2 | 5.87 | 11.8 | 14.1 |
| Fasted State Simulated Intestinal Fluid Crystal (FaSSIF) | 12.0 | 20.4 | 20.5 |
| Fed State Simulated Intestinal Fluid Crystal (FeSSIF) | 222 | 1145 | 1381 |

### (Test Example 5) Evaluation of Chemical Stability

### (Test Method)

The Form I crystal and Form II crystal were accurately weighed in aluminum pans and stored for 14 days under conditions of dry heat (60°C, 0% RH) and wet heat (40°C, 75% RH). The amount of increase in chemical analogs was measured under the same HPLC conditions as those used in the solubility test of Test Example 4.

### (Measurement Results)

As shown in Table 7, the Form I crystal was observed to demonstrate an increase in chemical analogs of 0.4% and 0.3%, respectively, while the Form II crystal was observed to demonstrate an increase in chemical analogs of 0.9% and 0.3%, respectively, under conditions of dry heat and wet heat. The increases in analogs were only slight and the crystals were stable.

**[Table 7]**

| Storage Condition | Increase in analogs (%) | |
|---|---|---|
| | Form I crystal | Form II crystal |
| 60°C/0% RH, 2 weeks | +0.4 | +0.9 |
| 40°C/75% RH, 2 weeks | +0.3 | +0.3 |

### (Test Example 6)

Male, 9-week-old, spontaneously diabetic rats (ZDF-Lepr^{fa}/CrlCrlj) were used.

A crystal of the compound of the present invention in the form of the Form I crystal at 10 mg/kg or the free form thereof in the form of Compound II at 8.3 mg/kg (calculated based on the free form, corresponding to the equivalent of 10 mg/kg of the Form I crystal) was respectively suspended in a 20% aqueous HP-β-cyclodextrin solution (hereinafter referred to as "vehicle") and orally administered under fasting conditions.

Blood glucose levels were measured in accordance with ordinary methods before administration of the compounds (at 0 hour) and at 0.5, 1, 2, 4 and 6 hours after administration. Namely, the tips of the rat tails (about 1 mm) were severed and blood collected with hematocrit tubes subjected to anticoagulation treatment with heparin were centrifuged followed by measuring the resulting plasma with the Glucoroder F (A&T Corporation). The areas under blood glucose curves from 0 to 6 hours after administration were calculated using the resulting blood glucose values.

The results of a blood glucose lowering activity test on each group administered with the Form I crystal, Compound II or vehicle only were as shown in Table 8.

Furthermore, the values shown in Table 8 are all the average values of test result values obtained using five spontaneously diabetic rats (ZDF-Lepr^{fa}/CrlCrlj).

**[Table 8]**

| | Area under blood glucose curve (mg/dl × hr) | P value (t-test) | |
|---|---|---|---|
| | | vs. vehicle group | vs. Compound II group |
| Vehicle dose group | 918.1 ± 52.3 | | |
| Compound II | 661.8 ± 26.9 | 0.0024 | |
| Form I crystal | 529.0 ± 24.5 | <0.0001 | 0.0065 |

Based on the results shown in Table 8, the crystal of the compound of the present invention in the form of the Form I crystal demonstrated blood glucose lowering activity that was superior to that of the free form thereof in the form of Compound II.

The Form I crystal and Form II crystal have superior solubility and are extremely stable, demonstrate high blood concentrations in comparison with Compound II in an evaluation of in vivo absorption, and have superior properties as a pharmaceutical crystal. Although there are both stable crystals in the form of the Form I crystal and metastable crystals in the form of the Form II crystal, both crystal forms can be selectively obtained by selecting a crystallization solvent.

| Preparation Example 1: Capsule | |
|---|---|
| Compound of Example 1 or 2 | 50 mg |
| Lactose | 128 mg |
| Cornstarch | 70 mg |
| Magnesium stearate | 2 mg |
| | 250 mg |

Powders of the above formulation were mixed and passed through a 60 mesh sieve followed by filling the powders into a 250 mg gelatin capsule to obtain a capsule.

| Preparation Example 2: Tablet | |
|---|---|
| Compound of Example 1 or 2 | 50 mg |
| Lactose | 126 mg |
| Cornstarch | 23 mg |
| Magnesium stearate | 1 mg |
| | 200 mg |

Powders of the above formulation were mixed, granulated using cornstarch paste and dried, followed by forming into tablets with a tableting machine to obtain a 200 mg tablet. This tablet can be provided with a sugar coating as necessary.

### INDUSTRIAL APPLICABILITY

According to the present invention, a crystal of a phenylpyrrole derivative can be provided that has superior solubility, hygroscopicity and stability. Crystals of a sulfonate of the phenylpyrrole derivative of the present invention are useful as pharmaceuticals.

## Claims

1. (2S)-2-(3-{5-[(5S)-5-Methyl-4,5-dihydro-1,3-oxazol-2-yl]-1H-pyrrol-2-yl}-5-{[5-(methylsulfonyl)pyrazin-2-yl]oxy}phenoxy)propan-1-ol methanesulfonate.

2. (2S)-2-(3-{5-[(5S)-5-Methyl-4,5-dihydro-1,3-oxazol-2-yl]-1H-pyrrol-2-yl}-5-{[5-(methylsulfonyl)pyrazin-2-yl]oxy}phenoxy)propan-1-ol monomethanesulfonate represented by the following formula (I):

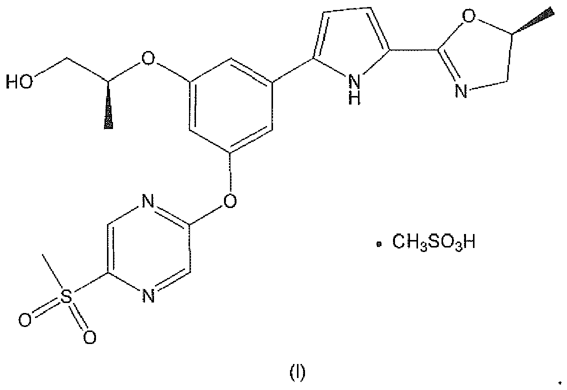

3. A crystal of (2S)-2-(3-{5-[(5S)-5-methyl-4,5-dihydro-1,3-oxazol-2-yl]-1H-pyrrol-2-yl}-5-{[5-(methylsulfonyl)pyrazin-2-yl]oxy]phenoxy)propan-1-ol monomethanesulfonate represented by the following formula (I) according to claim 2:

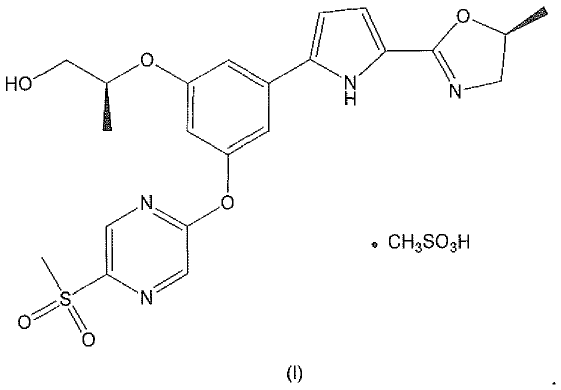

4. The crystal according to claim 3, wherein the crystal has characteristic peaks at 8.2, 17.1, 18.0, 19.5, 19.6, 20.0, 20.2, 22.0, 22.2, 23.5 and 24.1 (degrees) (each +0.2) for the angles of diffraction (2θ) as determined by powder X-ray diffraction obtained by irradiating with Copper Kα radiation.

5. The crystal according to claim 3, wherein the crystal has the characteristic peaks and relative intensities (angles of diffraction: ±0.2 each) shown in the following table:
**[Table 1]**
| Angle of diffraction 2θ (degree) | Interplanar spacing d (Å) | Relative intensity (%) |
|---|---|---|
| 8.2 | 10.8 | 15.1 |
| 17.1 | 5.2 | 15.7 |
| 18.0 | 4.9 | 15.5 |
| 19.5 | 4.6 | 28.8 |
| 19.6 | 4.5 | 17.2 |
| 20.0 | 4.4 | 22.2 |
| 20.2 | 4.4 | 100 |
| 22.0 | 4.0 | 42.7 |
| 22.2 | 4.0 | 20.6 |
| 23.5 | 3.8 | 25.1 |
| 24.1 | 3.7 | 15.4 |
for the angles of diffraction (2θ) as determined by powder X-ray diffraction obtained by irradiating with Copper Kα radiation.

6. The crystal according to claim 3, wherein the crystal has the characteristic peaks indicated by the pattern shown in the following diagram for the angles of diffraction (2θ) as determined by powder X-ray diffraction obtained by irradiating with Copper Kα radiation.

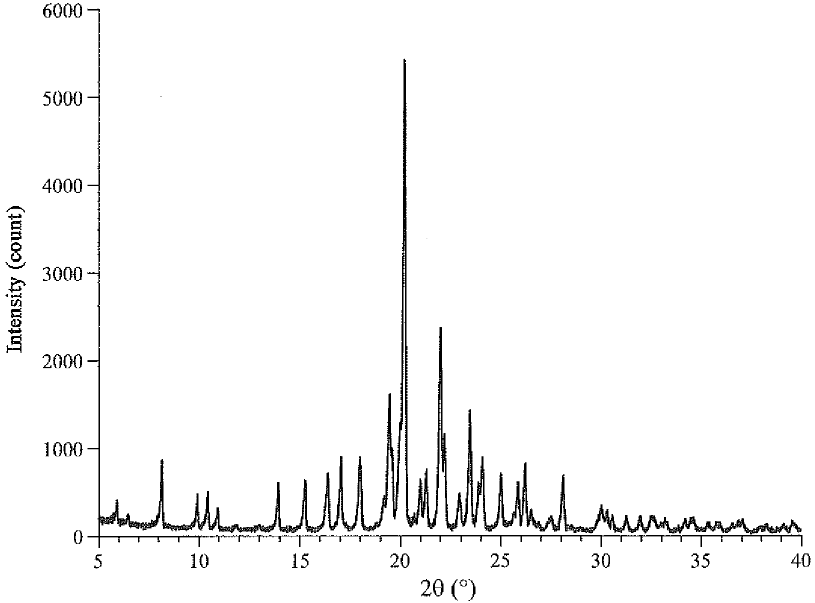

7. The crystal according to claim 3, wherein the crystal has at least one endothermic peak at 185°C to 195°C in differential thermal analysis (DTA).

8. The crystal according to claim 3, wherein the crystal has the characteristic peaks indicated by the pattern shown in the following diagram as a thermogravimetry-differential thermal analysis (TG/DTA) profile.

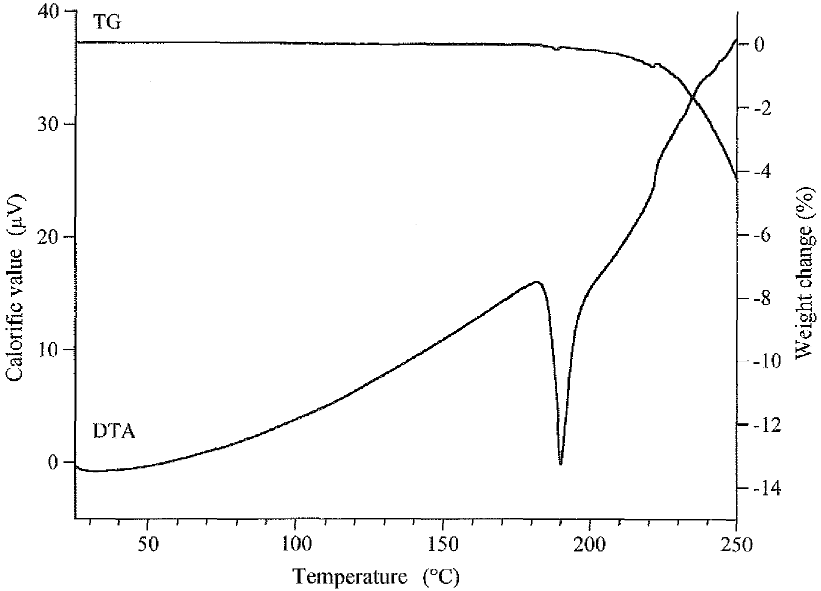

9. The crystal according to claim 3, wherein the crystal has characteristic peaks at 9.8, 15.9, 16.8, 18.2, 19.1, 19.7, 20.5, 22.3, 22.8, 23.4, 23.8, 24.6, 25.4, 25.6 and 27.8 (degrees) (each ±0.2) for the angles of diffraction (2θ) as determined by powder X-ray diffraction obtained by irradiating with Copper Kα radiation.

10. The crystal according to claim 3, wherein the crystal has the characteristic peaks and relative intensities (angles of diffraction: ±0.2 each) shown in the following table:
**[Table 2]**
| Angle of diffraction 2θ (degree) | Interplanar spacing d (Å) | Relative intensity (%) |
|---|---|---|
| 9.8 | 9.0 | 15.3 |
| 15.9 | 5.6 | 31.2 |
| 16.8 | 5.3 | 18.4 |
| 18.2 | 4.9 | 100 |
| 19.1 | 4.6 | 53.7 |
| 19.7 | 4.5 | 26.1 |
| 20.5 | 4.3 | 26.4 |
| 22.3 | 4.0 | 22.9 |
| 22.8 | 3.9 | 42.6 |
| 23.4 | 3.8 | 31.7 |
| 23.8 | 3.7 | 29.3 |
| 24.6 | 3.6 | 16.2 |
| 25.4 | 3.5 | 28.5 |
| 25.6 | 3.5 | 47.6 |
| 27.8 | 3.2 | 26.4 |
for the angles of diffraction (2θ) as determined by powder X-ray diffraction obtained by irradiating with Copper Kα radiation.

11. The crystal according to claim 3, wherein the crystal has the characteristic peaks indicated by the pattern shown in the following diagram for the angles of diffraction (2θ) as determined by powder X-ray diffraction obtained by irradiating with Copper Kα radiation.

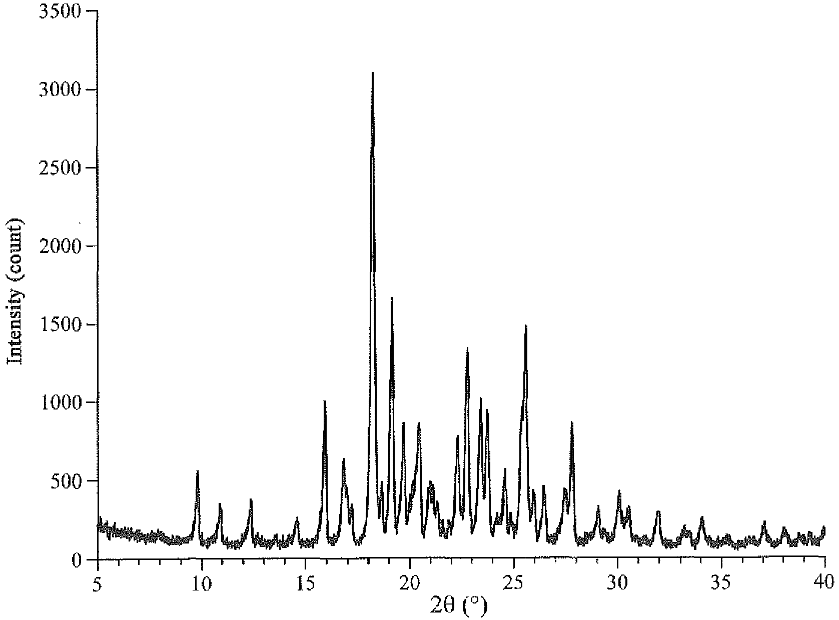

12. The crystal according to claim 3, wherein the crystal has at least one endothermic peak at 175°C to 185°C in differential thermal analysis (DTA).

13. The crystal according to claim 3, wherein the crystal has the characteristic peaks indicated by the pattern shown in the following diagram as a thermogravimetry-differential thermal analysis (TG/DTA) profile.

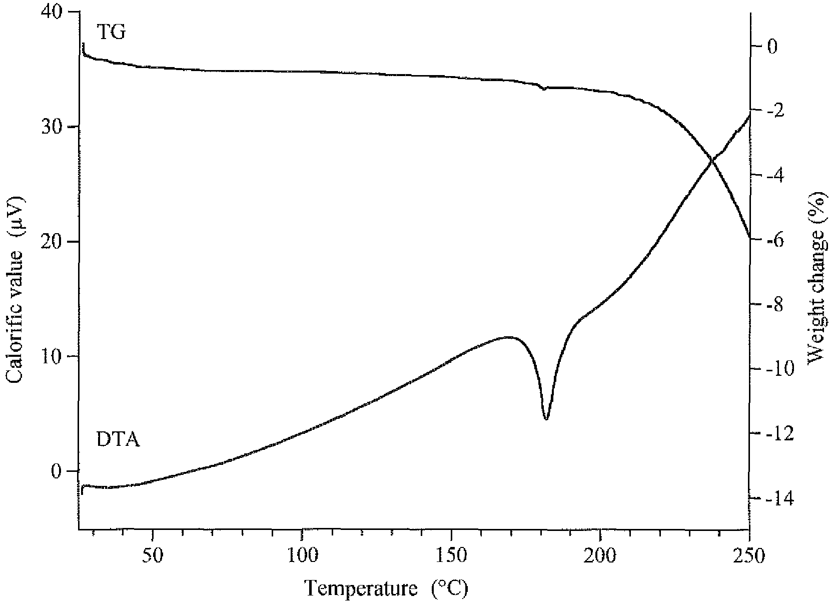

14. A pharmaceutical composition containing as an active ingredient thereof a compound according to claim 1 or 2 or a crystal according to any one of claims 3 to 13.

15. A pharmaceutical composition containing as an active ingredient thereof a crystal according to any one of claims 4 to 8 and/or a crystal according to any one of claims 9 to 13.

16. The pharmaceutical composition according to claim 14 or 15, wherein the pharmaceutical composition is for treating and/or preventing diabetes or impaired glucose tolerance.

17. A preventive drug or therapeutic drug for diabetes or impaired glucose tolerance containing as an active ingredient thereof a compound according to claim 1 or 2 or a crystal according to any one of claims 3 to 13.

18. Use of a compound according to claim 1 or 2 or a crystal according to any one of claims 3 to 13, for a preventive drug or therapeutic drug for diabetes or impaired glucose tolerance.

19. A method for producing a compound according to claim 2, **characterized by** reacting methanesulfonic acid with (2S)-2-(3-{5-[(5S)-5-methyl-4,5-dihydro-1,3-oxazol-2-yl]-1H-pyrrol-2-yl)-5-{[5-(methylsulfonyl)pyrazin-2-yl]oxy}phenoxy)propan-1-ol in a solvent.

20. The method according to claim 19, wherein the solvent is aqueous acetone.

21. The method according to claim 19, wherein the solvent is aqueous 1-propanol.
